# EUROPÄISCHE PATENTSCHRIFT

(11) **EP 0 223 049 B1**
(45) Veröffentlichungstag und Bekanntmachung des Hinweises auf die Patenterteilung: **29.12.1993**
(21) Anmeldenummer: 86114059.8
(22) Anmeldetag: 10.10.1986
(51) Int. Cl.: A61B 5/04, G06F 15/20, A61B 5/0402

(54) **Verfahren und Vorrichtung zur Darstellung elektrokardiografischer Werte**
Method and apparatus for displaying electrocardiographic data
Procédé et dispositif de représentation de grandeurs électrocardiographiques

(30) Priorität: 15.10.1985 DE 3536658
(43) Veröffentlichungstag der Anmeldung: 27.05.1987
(73) Patentinhaber: KESSLER, Manfred, Prof. Dr., D-91054 Erlangen (DE)
(72) Erfinder: KESSLER, Manfred, Prof. Dr., D-91054 Erlangen (DE)
(74) Vertreter: Seiffert, Klaus, Dipl.-Phys.

(56) Entgegenhaltungen:
- IEE PROCEEDINGS, Band 129, Nr. 5, Juli 1982, Seiten 340-351, Teil A, Old Woking, Surrey, GB; P. MANZONI et al.: "Electrocardiomultigraphimeter as a basic instrument for developing effective personal computing in cardiology"
- COMPUTERS IN CARDIOLOGY, Rotterdam, 2.-4. Oktober 1975, Seiten 241-244; C. ZEELENBERG et al.: "Results of a computerized system for cardiac catheterization"
- PHILIPS TECHNISCH TIJDSCHRIFT, Band 21, Nr. 1, 1959, Seiten 25-39, Eindhoven, NL; G.C.E. BURGER et al.: "Vector-elektrocardiografie"
- JAEU - JOURNAL OF THE ASIA ELECTRONICS UNION, Band 3, Nr. 3, 1970, Seiten 40-41, Tokyo, JP; Y. CHIYOSHI et al.: "Animation of electrocardiographic distribution on chest walls in color"
- IEEE TRANSACTIONS ON BIOMEDICAL ENGINEERING, Band BME-29, Nr. 5, Mai 1982, Seiten 322-332, IEEE, New York, US; G.E. MAILLOUX et al.: "Theoretical evaluation of the McFee and Frank vectorcardiographic lead systems using a numerical inhomogeneous torso model"
- "Anatomie, Physiologie und Pathophysiologie" von Thews et al, 1982, Seiten 198-199; Wissenschatl. Verlaggesellschaft mbH. Stuttgart

## Beschreibung

Die Erfindung betrifft ein Verfahren zur Darstellung elektrokardiographischer Werte, die durch Ableitelektroden abgeleitet und durch Verstärker und Anzeigemittel anzeigbar sind.

Ebenso betrifft die vorliegende Erfindung eine Vorrichtung zur Durchführung eines solchen Verfahrens, welche frontal und dorsal anzuordnende Ableitelektroden, Meßeinrichtungen und Verstärker für die zwischen den Ableitelektroden gemessenen Potentialwerte und eine Anzeigevorrichtung aufweist. Eine solche Vorrichtung ist allgemein als Elektrokardiographiegerät (EKG-Gerät) bekannt. Entsprechende Verfahren sind allgemein als Elektrokardiographie bekannt.

Die Elektrokardiographie ist eine bedeutsame Methode der Messung der Herzaktivität durch Messung der bei der Bewegung des Herzmuskels entstehenden elektrischen Potentiale.

Die Ausdeutung der elektrokardiographisch gewonnenen Werte, die meist als kontinuierlich verlaufende Kurven vorliegt, ist zwar eine etablierte Diagnosemethode, hat jedoch bisher den Nachteil, daß die Kurven einen mehr oder weniger abstrakten Zusammenhang mit der Kontraktion des Hohlmuskels zeigen. Das liegt daran, daß das elektrische Signal dann am größten ist, wenn der vom Muskel gebildete elektrische Dipol seinen Maximalwert, nämlich alle Fasern des Muskels erreicht hat. Dieser Zustand liegt meist vor, wenn die räumliche Ausbreitung der Kontraktion bis zur Mitte des Muskels fortgeschritten ist. Sie weist dort den prozentual stärksten Zuwachs auf.

Das Aktivitätssignal entspricht also etwa dem Differentialquotienten der Kontraktion. Wünschenswert wäre aber eine Größe, die der räumlichen Ausbreitung der Kontraktion entspricht.

Ein Beispiel für den hohen Informationsgehalt einer Messung der elektrischen Herzaktivität und gleichzeitig schwieriger Deutbarkeit ist das Vektorelektrokardiogramm.

Eine Übersicht über die Vektor-Elektrokardiographie findet man in Philips Technisch Tijdschrift 21 (1951) 1, Seiten 25-39.

Computerberschnungen über Betrags- und Richtungsänderungen des mit dem Herzschlag verknüpften Dipolvektors werden in den IEE Proc. Vol. 129, Nr. 5, 1982, Seiten 340 - 350, berichtet. Eine Vorstellung über die Verknüpfung der Form eines EKG-Signales mit der Lage von erregten Herzmuskelzellen und der Ausbreitung einer entsprechenden Erregungsfront ist in dem Buch von Thews et al "Anatomie Physiologie Pathophysiologie des Menschen", Wissenschaftliche Verlagsgesellschaft mbH Stuttgart, 1982, auf den Seiten 198 und 199 zu finden.

Alle vorgenannten Druckschriften haben grundsätzlich nichts an der Situation geändert, daß für die Diagnose von Herzkrankheiten nach wie vor die komplizierten Kurvenverläufe, die man aus den bekannten EKG-Geräten erhält, interpretiert werden müssen, wobei dies Schwierigkeiten im Falle von Elektrokardiogrammen wegen der höheren Komplexität der Kurven noch schwieriger sind. Generell erfordert die Deutung von Elektrokardiogrammen eine große Erfahrung, um Herzkrankheiten und Herzfehler auch bei nur kleinen Abweichungen von der theoretischen, idealen EKG-Kurve zuverlässig diagnostizieren zu können.

Demgegenüber liegt der vorliegenden Erfindung die Aufgabe zugrunde, mit Hilfe von EKG-Messungen eine Darstellung der Aktivität des Herzmuskels zu geben, die einerseits nur von den gemessenen elektrischen Potentialen abhängt, andererseits aber direkt ein Bild der räumlichen Ausbreitung der Kontraktion im Herzmuskel ergibt.

Hinsichtlich des Verfahrens wird diese Aufgabe durch ein Verfahren mit den Merkmalen des Anspruches 1 gelöst, hinsichtlich der zugehörigen Vorrichtung durch eine Vorrichtung mit den Merkmalen des Anspruches 10. Durch das erfindungsgemäße Verfahren werden also aus den gemessenen elektrischen Potentialen ein oder mehrere, die elektrischen Aktivitäten im Herzen repräsentierende Dipole ermittelt. Gleichzeitig wird gemäß der vorliegenden Erfindung die Erregungsfront als zu dem Dipol bzw. den Dipolen senkrechte Fläche dargestellt und diese Fläche wird in einem Herzmodell auf einer Anzeigeeinrichtung als durch das Herz verlaufende Erregungsfront wiedergegeben.

Diese apparative Eintragung der Schichtlinien und der Schichtflächen in da grafische Herzmodell erlaubt die einfache Beobachtung und Beurteilung der Erregungsausbreitung im Herzmuskel.

Die Vorrichtung zur Erstellung dieser Darstellung besteht aus den Ableitelektroden und zugeordneten, mit Anzeigemitteln versehenen Verstärkern, wobei die Signale der Ableitelektroden einem Rechenwerk digital zugeführt sind, das daraus die Koordinaten des Erregungsablaufes berechnet, anschließend die errechneten Koordinaten einer Anzeigevorrichtung zuleitet, auf der gleichzeitig ein grafisches Herzmodell permanent dargestellt ist.

Im einzelnen ist das Herzmodein als dreidimensionales Drahtmodell oder Umrißmodell dargestellt, In das die Schichtlinien oder Schichtflächen der Erregungsausbreitung eingetragen werden, deren räumliche Lage durch den jeweils berechneten elektrischen Hauptvektor und deren Abstand zueinander durch die Änderung des Betrages des Hauptvektors bestimmt ist.

Hierdurch wird der gesamte Inhalt der fuer ein Vektorkardiogramm erforderlichen Messungen verwertet, jedoch in einer Form dargestellt, die den Erregungsablauf unmittelbar erkennen laesst.

Bei besonders komplizierten Ablaeufen oder zu Demonstrationszwecken kann es von Vorteil sein, wenn das Herzmodell in mehreren zweidimensionalen Schnitten dargestellt ist.

In Weiterentwicklung des Verfahrens werden die Signale der Ableitelektroden auf einem Speichermedium, beispielsweise einer Magnetplatte gespeichert, sind von dort durch das Rechenwerk abrufbar und nach Berechnung der Darstellungskoordinaten auf einem Anzeigegeraet darstellbar.

Der Vorteil dieser Weiterentwicklung liegt einmal darin, dass nunmehr ein off-line Betrieb der Anzeigevorrichtung ermoeglicht ist, der geringere Rechengeschwindigkeiten zur Berechnung der Schichtlinien oder Schichtflaechen benoetigt und dass es weiterhin gelingt, die Erregungsausbreitung entweder in Zeitlupe zu verfolgen, indem die langsame Entstehung der Schichtebenen oder Schichtlinien der Erregungsausbreitung betrachtet wird, oder das Schichtlinienmodell als ganzes zu begutachten, wobei der Abstand der Schichtlinien den Zeitablauf charakterisiert und wobei die Neigung der Schichtebenen der Richtung des Hauptvektors entspricht.

Dabei zeigt sich die grosse Ueberlegenheit der direkt anzeigenden Methode gegenueber den abstrakteren Methoden der herkoemmlichen Elektro-oder Vektorkardiogramme.

Anstelle einer isovolumetrischen Darstellung mit Schichtlinien oder Schichtebenen kann der Kontraktionszustand auch durch Farbfuellung des Herzmodells angezeigt werden, indem ein Hauptvektor berechnet wird und der Hauptvektor den Fortlauf der Farbausfuellung des Modells bestimmt. Diese Darstellung eignet sich besonders fuer zweidimensionale Schnitte.

Bei Darstellung von Schichtflaechen werden zweckmaessig verschiedene Farben verwendet, um den Erregungsablauf in den Atrien vom Erregungsablauf in den Ventrikeln zu unterscheiden. Auch bei der Darstellung der Schichtflaechen selbst lassen sich zur Verbesserung der Unterscheidung von Unterseite und Oberseite der Schichtflaechen verschiedene Farben oder Schraffuren verwenden.

Eine verbesserte Darstellung des Erregungsablaufs ist dadurch erreichbar, dass jedem anatomischen Element des Modells ein Vektor zugeordnet ist, weil damit zusaetzliche Einzelheiten des Kontraktionsablaufes gewonnen und dargestellt werden koennen.

Voraussetzung dazu ist jedoch eine entsprechend spezifizierte Ableitung der Aktionspotentiale. Sie kann durch unmittelbar am Muskel angeordnete Ableitelektroden gewonnen werden.

Sie laesst sich aber auch ueber zwei, jeweils frontal und dorsal angeordnete, als "Rasterelektroden" bezeichnete Netze von Ableitelektroden bestimmen, die vorzugsweise von der elektrischen Herzachse durchstossen werden und wobei die Elektroaktivitaet zwischen je einer Frontal-und Dorsalelektrode der beiden Rasterelektroden nacheinander oder gleichzeitig gemessen wird.

Bei einer daraus abgeleiteten Darstellung muss dann jedem Elektrodenpaar ein Einzelvektor zugeordnet sein. Dadurch kann nicht nur eine idealisierte zweidimensionale Schichtflaeche als Ausbreitungsfront dargestellt werden, sondern die Schichtflaeche wird weiter aufgeloest und es entsteht eine dreidimensionale Schichtflaeche.

Eine andere Weiterentwicklung der Erfindung besteht darin, dass die anatomischen Elemente des Modells, wie beispielsweise die raeumliche Ausdehnung der Vorhoefe und Kammern, das Muskelvolumen, der Ort von Erregungssenken oder von Erregungsquellen raeumlich und/oder zeitlich im Modell einstellbar sind. Dadurch ist es moeglich, das Muskelmodell als Phantommodell des Herzmuskels auszubilden, dieses jedoch den tatsaechlich gemessenen Gegebenheiten anzupassen.

Aus der Wahl der Parameter, die sich programmtechnisch als Amplituden, Frequenzen, Phasen, Zeitverzoegerungen oder zeitliche Spruenge darstellen lasssen, koennen Aussagen ueber die anatomischen Verhaeltnisse am realen Muskel gewonnen werden.

Eine weitere Anpassung an den tatsaechlichen Erregungsablauf kann dadurch erfolgen, dass das elektrische Modell zusammen mit einem tatsaechlichen Bild des Herzmuskels auf der selben Anzeigevorrichtung darstellbar ist. Dadurch koennen ebenfalls die Modellparameter an den realen Muskel angepasst werden.

In der Zeichnung, anhand derer die Erfindung erlaeutert wird, zeigen:
Fig.1a-c Ein Modell der Messanordnung mit Ableitpunkten und Darstellungen des Herzmodells,
Fig.2a-c Rasterelektroden zur genaueren messtechnischen Bestimmung von Erregungsfronten und Hauptvektoren.

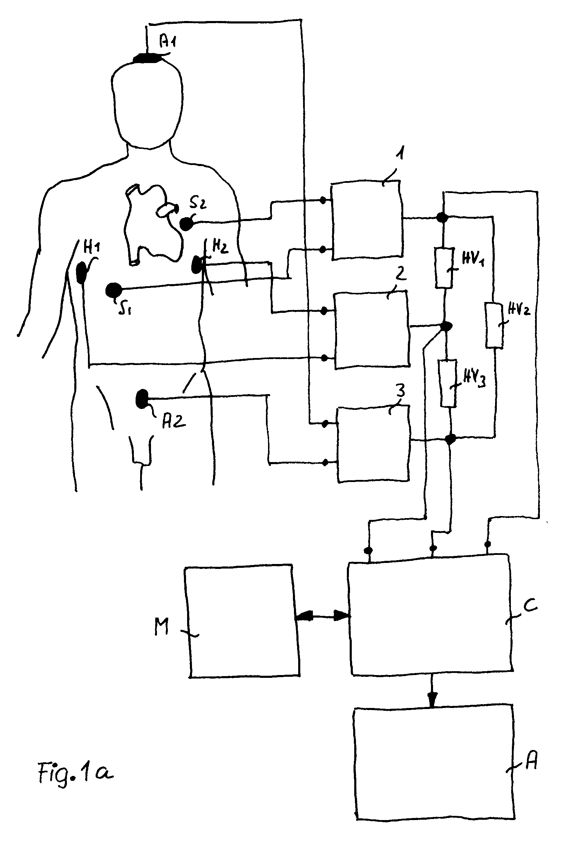

In Fig.1a sind die Ableitpunkte A1,A2,S1,S2,H1,H2 dargestellt, die im vorliegenden Beispiel zur Ableitung der Elektroaktivitaet verwendet werden koennen und im stand der Technik ueblich sind. Es koennen dabei auch andere der bekannten Ableitungen verwendet werden, wenn entsprechende Rechenroutinen in einem zugeordneten Rechner C zur Vektorbestimmung zur Verfuegung gestellt werden.

Im Beispielsfall sind A1,A2 die axialen, H1,H2 die horizontalen und S1,S2 die sagittalen Elektroden. Ihre durch Verstaerker 1,2,3 verstaerkten Signale werden in der bisher bekannten Weise als orthogonale Vektoren HV1,HV2,HV3 betrachtet und im elektronischen Rechner C zu einem Hauptvektor HV zusammengesetzt.

Nach der Erfindung wird dieser Hauptvektor jedoch nicht mehr als Vektor allein oder als Endpunkt zweidimensional dargestellt, sondern er dient als Normale einer Schichtflaeche, beispielsweise der Schichtflaeche SF1 in einem auf einem Sichtschirm A permanent dargestellten Herzmodell HM, Fig.1b.

Die Darstellung der Normalen selbst kann dabei auch entfallen, wenn anstelle des Vektorbetrages beispielsweise der Abstand Ui der Schichtflaechen eingestellt wird. Es ergibt sich dann ein Schichtlinienbild aus diesen jeweils aufzuzeichnenden Schichtflaechen SF1,SF2,..SFi, deren Abstaende U1,U2,.. durch eben diesen Betrag des jeweiligen Hauptvektors HV bestimmt sind.

Die Darstellung der Schichtflaechen (SF1,SF2,..) laesst sich dadurch verbessern, dass die jeweils sichtbare Flaeche durch besondere Flaechenmuster als Oberseite oder Unterseite gekennzeichnet wird.

Bei besonders komplizierten Vorgaengen koennen auch einzelne Flaechenschnitte HM2 in einem Herzmodell nach Fig.1c dargestellt und die gemessenen oder berechneten Erregungsfronten (SF1, SF2..) in das Modell eingetragen werden.

In jedem Falle werden die messtechnisch oder rechnerisch gewonnenen Werte in einem Datenspeicher M des Rechners C abgelegt.

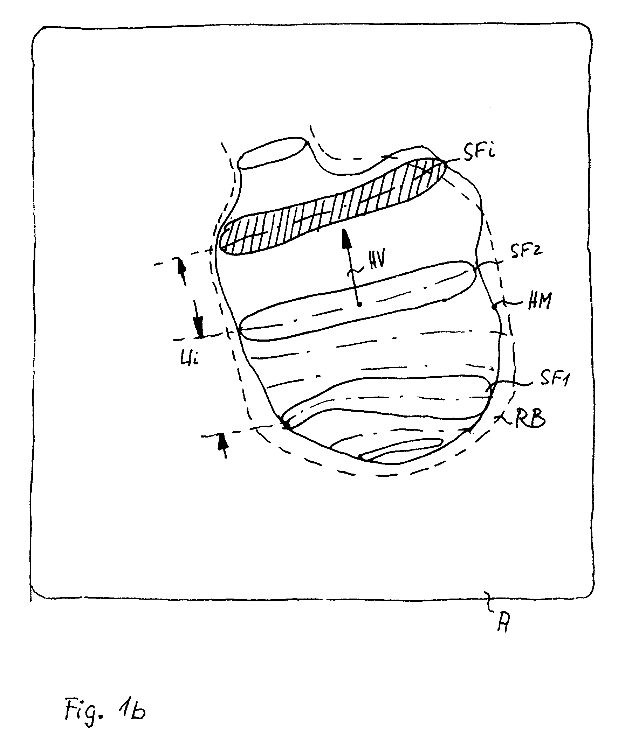

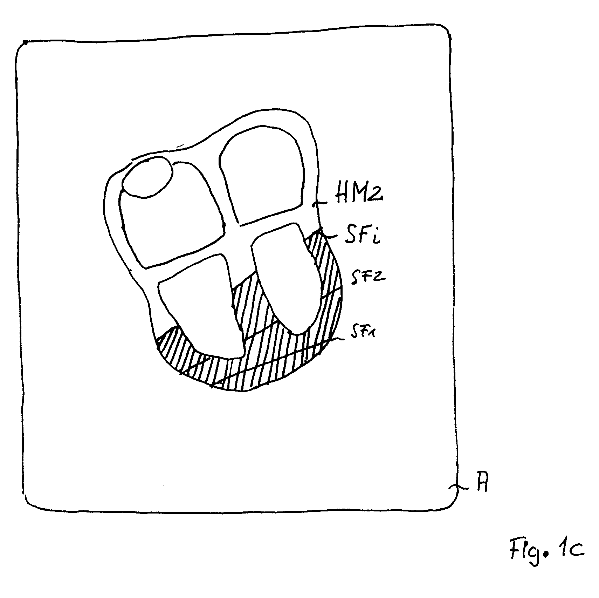

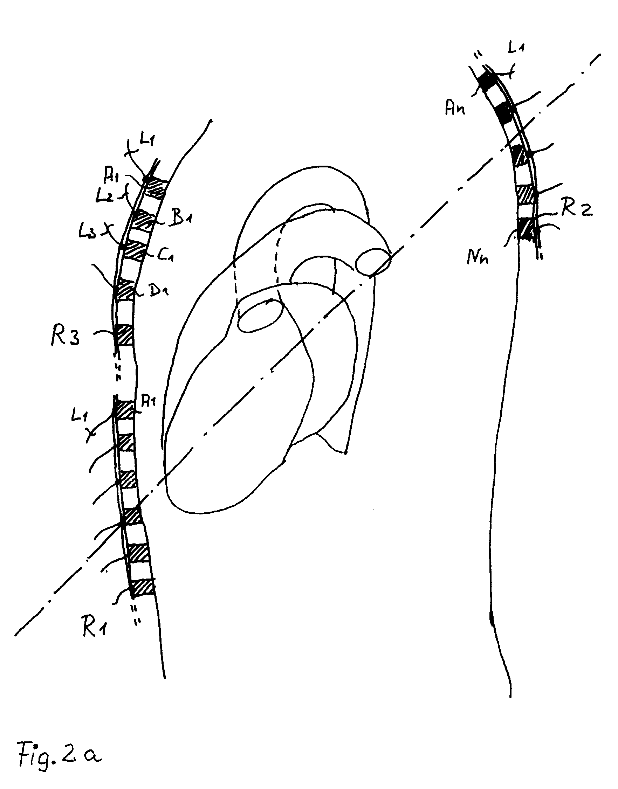

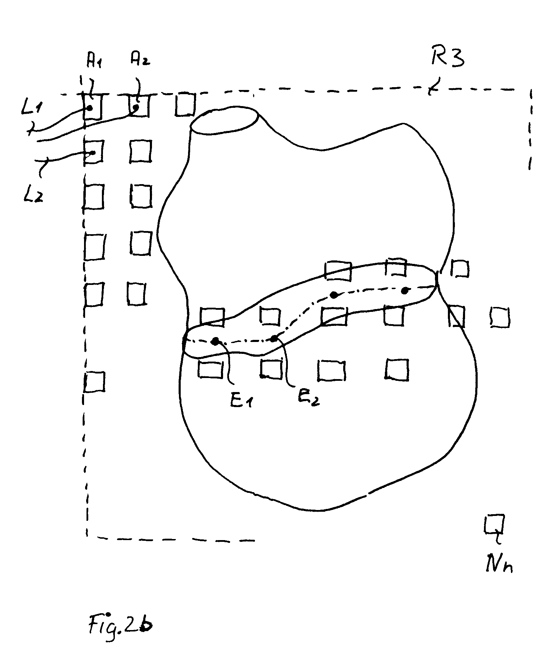

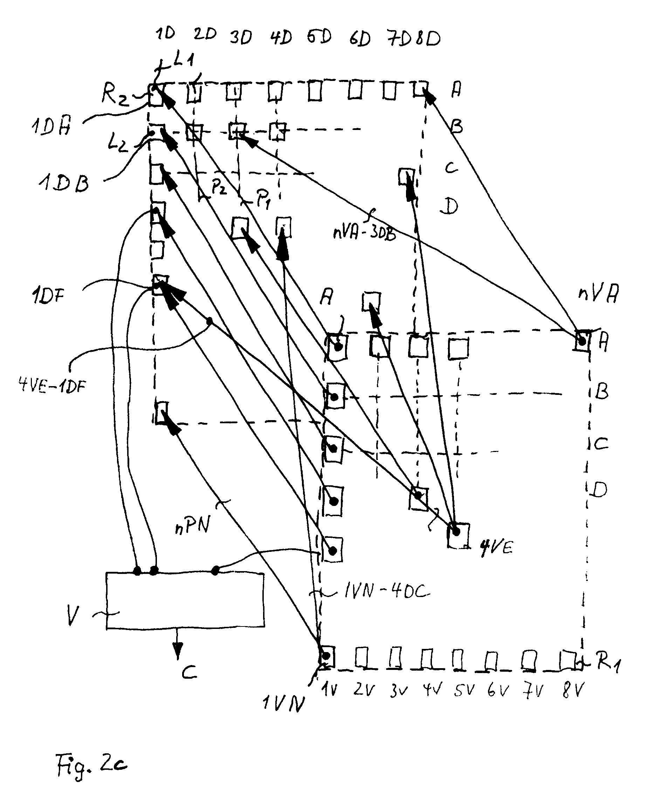

Eine Verbesserung der Darstellung laesst sich erzielen, wenn nach Fig.2 ein als "Rasterelektrode" R1, R2, R3 zusammengefasstes Netz von Ableitelektroden A1,A2..Nn frontal und/oder dorsal ueber dem Herzmuskel angebracht wird.

Bei frontaler Anbringung einer einzigen Rasterelektrode (R3) auf einer Flaeche maximaler Projektion des Herzmuskels auf der Brustwand kann die bekannte unipolare WILSON-Ableitung angewendet werden. Dabei werden die Elektroden A1,A2..Nn, die mit Leitungen L1, L2.. mit den (nicht gezeichneten) Verstaerkern verbunden sind, gegen die (nicht gezeichnete) Sammelelektrode gemessen, so dass gemaess Fig.2b durch Aufsuchen der jeweils maximalen Signale aus den A1.. Nn Elektroden der Verlauf der Erregungsfront E1,E2,... bestimmt werden kann.

Werden zwei Rasterelektroden R1,R2 nach Fig.2 frontal und dorsal so angeordnet, dass die Herzachse durch die Rasterelektroden verlaeuft, dann koennen gemaess Fig.2c die verschiedenen Schnittvektoren bestimmt werden:
der Herzachse parallele "Parallelvektoren"
1PA....1PN
oder "Buendelvekoren"
4VE-1DF (zentraler Buendelvektor)
nVA-3DB (rechtsapikaler Buendelvektor)
1VN-4DC (linksbasaler Buendelvektor)
Aus diesen Einzelvektoren kann der jeweils vorliegende Maximalvektor durch Selektion der Signale aus allen Messkanaelen des Verstaerker V durch den Rechner C ermittelt werden.

Da die Muskelaktivitaet im wesentlichen in Richtung der Herzachse verlaeuft, kann mit diesem Verfahren bereits eine gute messtechnisch bestimmte Naeherung an den tatsaechlichen Ablauf der Erregungsausbreitung gefunden werden.

Die Messung selbst wird dabei durch den elektronischen Rechner C gesteuert und in Intervallen vorgenommen, die klein gegen den Gesamtzyklus des Muskels sind. Dabei werden die digitalen Werte der Elektrodensignale zur Berechnung des Hauptvektors HV verwendet, oder der Rechner C nimmt die Selektion des Maximalvektors vor. Die gewonnenen Daten werden dabei vorzugsweise in dem Datenspeicher M des Rechners C abgelegt.

Die erfindungsgemaesse Darstellungsweise laesst sich erweitern. In Fig.1b sind zwei Momentaufnahmen auf der selben Anzeigevorrichtung A abgebildet: Ein Bild des Herzphantoms HM und ein Umrissmodell RB des realen Muskels. Dabei ist das reale Bild RB beispielsweise durch eine Kamera bei der Durchleuchtung mit Roentgenlicht aufgenommen, das Phantombild HM ist durch den Rechner C intern erzeugt.

Wird eine Folge von mehreren realen Bildern RB und Herzmodellen HM hintereinander aufgenommen, dann laesst sich vergleichen, ob der Erregungsablauf im Phantombild HM und die Kontraktion im Realbild RB uebereinstimmen. Damit laest sich ein erster Anhalt gewinnen, wie die tatsaechliche Aktivitaetsverteilung im Herzmuskel ablaeuft, da die Grundlage fuer das Phantombild die Messung der tatsaechlichen elektrischen Herzaktivitaet ist.

## Patentansprüche

1. Verfahren zur Darstellung elektrokardiografischer Werte, die durch Ableitelektroden abgeleitet und durch Verstärker und Anzeigemittel anzeigbar sind, dadurch gekennzeichnet, daß die Signale der Ableitelektroden in Intervallen, die klein gegen den Gesamtzyklus des Herzmuskels sind, zeitlich aufgenommen, digitalisiert und einem Rechenwerk (C) zur Berechnung von die Erregungsausbreitung beschreibenden Koordinaten zugeleitet werden, daß diese Koordinaten rechnerisch zu Schichtflächen oder Schichtlinien (SF1, SF2...) ergänzt und als Abfolgen mit Schichtabständen (U1, U2...) in ein auf einer Anzeige (A) dargestelltes grafisches Herzmodell (HM) eingetragen werden, wobei der Herzmuskel als aus einem oder mehreren elektrischen Dipolen zusammengesetzt betrachtet wird und die Schichtflächen als Normalflächen zu dem Dipol bzw. den Dipolen gebildet werden, so daß der Erregungsablauf am Herzmodell (HM) erkennbar wird.

2. Verfahren nach Anspruch 1, dadurch gekennzeichnet, daß für die Ableitungen Rasterelektroden (A1, L1, ... Nn) frontal und dorsal so angeordnet werden, daß ihre gedachten Verbindungslinien durch da Herz verlaufen.

3. Verfahren nach Anspruch 2, dadurch gekennzeichnet, daß zu jeder frontalen Rasterelektrode (A1...) diejenige dorsale Elektrode (L1...) ermittelt wird, welche gegenüber der betreffenden frontalen Elektrode den maximalen Potentialabstand hat oder daß umgekehrt zu jeder dorsalen Elektrode (L1...) eine entsprechende frontale Elektrode (A1...) ermittelt wird, und daß die Verbindungslinien der Elektrodenpaare mit den maximalen Signalen die Lage und Richtung zugehöriger Dipolvektoren definieren und daß die Schichtflächen aus der Gesamtheit der zu den maximalen Dipolen gehörenden Normalflächen zusammengesetzt wird.

4. Verfahren nach Anspruch 1, dadurch gekennzeichnet, daß das Herzmodell in zweidimensionalen Schnitten (HM2) auf einer Anzeigevorrichtung (A) dargestellt ist.

5. Verfahren nach Anspruch 1, dadurch gekennzeichnet, daß die Signale der Ableitelektroden (A1, L1, ... Nn) digital gespeichert, und durch das Rechenwerk (C) abrufbar sind und daß nach Berechnung der Schichtflächen oder Schichtlinien (SF1, SF2...) diese zusammen mit einem Herzmodell (HM) auf einer Anzeigevorrichtung (A) darstellbar sind.

6. Verfahren nach Anspruch 1, dadurch gekennzeichnet, daß durch ein Vektorkardiogramm der Hauptdipolvektor des Herzens gemessen und die zugehörige Erregungsfront als zum Hauptvektor senkrechte Fläche dargestellt wird.

7. Verfahren nach einem der Ansprüche 1 bis 6, dadurch gekennzeichnet, daß die Lage der Flächen, weiche die von der Herzspitze ausgehende Erregungsfront repräsentieren, aus der Änderung des Betrages des jeweiligen Dipolvektors bestimmt wird, wobei die maximale Änderung des Dipolbetrages einer Lage derzugehörigen Erregungsfront in der Mitte des Herzens entspricht.

8. Verfahren nach einem der Ansprüche 1 bis 7, dadurch gekennzeichnet, daß das Herzmodell (HM) zusammen mit einem Durchleuchtungsbild (RB) auf derselben Anzeigevorrichtung darstellbar ist.

9. Verfahren nach Anspruch 8, dadurch gekennzeichnet, daß die Lage der zu einem Dipolvektor gehörenden Erregungsfront durch Vergleich mit dem Durchleuchtungsbild (RB) ermittelt wird.

10. Vorrichtung mit Mitteln zur Durchführung des Verfahrens nach einem der Ansprüche 1 bis 9, weiche in einer frontalen und einer dorsalen Anordnung anbringbare Ableitelektroden (A1, A2, H1, H2, H1, H2...) aufweist sowie Meßeinrichtungen und Verstärker zur Messung der Signale zwischen den Ableitelektroden und eine Anzeigevorrichtung aufweist, wobei die Meßeinrichtungen für eine Erfassung aller Paare von frontalen und dorsalen Elektroden ausgelegt sind, deren gedachte Verbindungslinien durch das Herz verlaufen und daß Speichereinrichtungen für die Speicherung der gemessenen Potentialwerte vorgesehen sind, daß die Anzeigevorrichtung ein Bildschirm ist und daß ein Rechenwerk zur Verarbeitung der gespeicherten Daten und deren anschauliche Darstellung auf dem Bildschirm vorgesehen ist.

## Claims

1. Method for representing electrocardiographic values, which are determined by sensing electrodes and can be indicated by means of amplifier and display means, characterised in that the signals from the sensing electrodes are periodically sensed in intervals which are small compared to the total cycle of the cardiac muscle, are digitised and fed to a computing means (C) for calculation of the coordinates which describe the spread of excitation, that these coordinates are computationally supplemented to layer surfaces or layer lines (SF1, SF2 etc) and fed as sequences with gaps between the layers (U1, U2 etc) to a heart model (HM) which is represented on a display means (A), whereby the cardiac muscle is considered to be composed of one or more electrical dipoles, and the layer surfaces are formed as normal surfaces to the dipole or dipoles so that the excitation sequence is recognisable on the heart model (HM).

2. Method according to claim 1, characterised in that for sensing, scanning electrodes (A1, L1 - Nn) are arranged frontally and dorsally in such a way that their theoretical connecting lines pass through the heart.

3. Method according to claim 2, characterised in that for each frontal scanning electrode (A1 etc) the dorsal electrode (L1 etc) is determined which has the maximum difference in potential with respect to the frontal electrode in question, or conversely, for each dorsal electrode (L1 etc) the corresponding frontal electrode (A1 etc) is determined, and that the connecting lines of the pairs of electrodes with the maximum signals define the location and the direction of the associated dipole vectors and that the layer surfaces are composed of the totality of the normal surfaces belonging to the maximum dipoles.

4. Method according to claim 1, characterised in that the heart model (HM) is represented in two-dimensional sections (HM2) on a display means (A).

5. Method according to claim 1, characterised in that the signals from the sensing electrodes (A1, L1 - Nn) are digitally stored and can be consulted by the computing means (C) and that after calculation of the layer surfaces or layer lines (SF1, SF2 etc) these can be represented together with a heart model (HM) on a display means (A).

6. Method according to claim 1, characterised in that the principal cardiac dipole vectors are measured by a vector cardiogram and the associated excitation front is represented as a surface perpendicular to the principal vector.

7. Method according to one of claims 1 to 6, characterised in that the location of the surfaces which are represented by the excitation front emanating from the tip of the heart are determined by the change of value of the respective dipole vector, whereby the maximum change of the dipole value corresponds to a location of the associated excitation front in the middle of the heart.

8. Method according to one of claims 1 to 7, characterised in that heart model (HM) can be represented together with a fluoroscopic heart model (HM) can be represented together with a fluoroscopic image (RB) on the same display means.

9. Method according to claim 8, characterised in that the location of excitation front associated with a dipole vector is sensed by comparison with the fluoroscopic image (RB).

10. Apparatus with means for carrying out the method according to one of claims 1 to 9, which is provided with sensing electrodes (A1, A2, H1, H2, H1, H2 etc) which can be applied in a frontal and dorsal arrangement, as well as measuring devices and amplifiers for measuring the signals between the sensing electrodes and a display means, whereby the measurement apparatus is set up for sensing all the pairs of frontal and dorsal electrodes, the theoretical connecting lines of which pass through the heart, and memory means for storing the potential values measured are provided, and the display means is a screen, and an computing means for processing the stored data and visibly representing it on the screen is provided.

## Revendications

1. Procédé pour représenter des valeurs électrocardiographiques, obtenues à partir d'électrodes de dérivation et pouvant être affichées par l'intermédiaire d'un amplificateur et d'un dispositif d'affichage, caractérisé en ce que les signaux des électrodes de dérivation sont enregistrés selon des intervalles de temps qui sont petits par rapport au cycle total du muscle cardiaque, puis sont numérisés et envoyés dans un organe de calcul (C), destiné à calculer les coordonnées qui décrivent la propagation de l'excitation ; que ces coordonnées sont, par le calcul, complétées pour donner des surfaces ou des lignes de niveau (SF1, SF2 ...) et, en suivant les écarts entre les lignes de niveau (U1, U2 ...), sont introduites dans un modèle graphique de coeur (HM), visualisé sur un dispositif d'affichage (A), le muscle cardiaque étant considéré comme constitué d'un ou plusieurs dipôles électriques, et les surfaces de niveau étant formées sous forme de surfaces normales au ou aux dipôles, de sorte que l'on peut reconnaître l'évolution de l'excitation sur le modèle cardiaque (HM).

2. Procédé selon la revendication 1, caractérisé en ce que, pour les dérivations, des électrodes à balayage (A1, L1, ... Nn) sont disposées, côté frontal et côté dorsal, de façon que leurs lignes de liaison virtuelles passent par le coeur.

3. Procédé selon la revendication 2, caractérisé en ce que, pour chaque électrode à balayage frontal (A1 ...) on définit l'électrode dorsale (L1 ...) qui présente par rapport à l'électrode frontale considérée l'écart de potentiel le plus grand, ou que, inversement, et pour chaque électrode dorsale (L1 ...), on définit une électrode frontale correspondante (A1 ...), et que les lignes qui relient les paires d'électrodes correspondant aux signaux maximaux définissent la position et la direction des vecteurs dipolaires correspondants, et que les surfaces de niveau sont constituées de la totalité des surfaces normales appartenant aux dipôles maximaux.

4. Procédé selon la revendication 1, caractérisé en ce que le modèle cardiaque est représenté sur un dispositif d'affichage (A) selon des coupes bidimensionnelles (HM2).

5. Procédé selon la revendication 1, caractérisé en ce que les signaux des électrodes de dérivation (A1, L1, ... Nn) sont mémorisés par un processus numérique, et peuvent être interrogés par l'organe de calcul (C), et que, après le calcul des surfaces ou des lignes de niveau (SF1, SF2 ...) ces dernières peuvent, avec un modèle cardiaque (HM) être visualisées sur un dispositif d'affichage (A).

6. Procédé selon la revendication 1, caractérisé en ce qu'on mesure le vecteur bipolaire principal du coeur par l'intermédiaire d'un cardiogramme vectoriel, et que l'on visualise le front d'excitation correspondant sous forme d'une surface perpendiculaire au vecteur principal.

7. Procédé selon l'une des revendications 1 à 6, caractérisé en ce que la position des surfaces qui représentent le front d'excitation partant de la pointe du coeur est déterminée à partir de la variation de la grandeur du vecteur bipolaire considéré, la variation maximale de la valeur du vecteur dipolaire correspondant à une position du front d'excitation considéré se trouvant au milieu du coeur.

8. Procédé selon l'une des revendications 1 à 7, caractérisé en ce que le modèle cardiaque (HM) est, avec une image radiographique (RB), représenté sur le même dispositif d'affichage.

9. Procédé selon la revendication 8, caractérisé en ce que la position du front d'excitation correspondant à un vecteur dipolaire est déterminée par comparaison avec l'image radiographique (RB).

10. Dispositif comportant des moyens pour mettre en oeuvre le procédé selon l'une des revendications 1 à 9, qui comporte des électrodes de dérivation (A1, A2, H1, H2, H1, H2 ...) pouvant être disposées selon une disposition frontale et une disposition dorsale, et qui comporte aussi des dispositifs de mesure et des amplificateurs pour mesurer les signaux entre les électrodes de dérivation et un dispositif d'affichage, dans lequel les dispositifs de mesure sont conçus pour capter toutes les paires d'électrodes frontales et dorsales, dont les lignes de liaison virtuelles passent par le coeur, et que l'on prévoit des dispositifs de mémorisation pour mémoriser les valeurs mesurées du potentiel, que le dispositif d'affichage est un écran et que l'on prévoit un organe de calcul pour traiter les données mémorisées et les représenter d'une manière claire sur l'écran.
